# EUROPEAN PATENT APPLICATION

(11) **EP 1 293 576 A1**
(43) Date of publication of application: **19.03.2003**
(21) Application number: 01402388.1
(22) Date of filing: 18.09.2001
(51) Int. Cl.: C12Q 1/68

(54) **Compositions and methods to identify haplotypes**

(71) Applicant: Integragen, 91000 Evry (FR)
(72) Inventor: Hager, Jörg, 91540 Mennecy (FR)
(74) Representative: Becker, Philippe

(57) **Abstract**

The present invention relates to the field of genomics and genetic analysis, more particularly to genetic mapping of complex quantitative and qualitative traits. This invention more particularly relates to compositions and methods to identify haplotypes of associated allelic variants in nucleic acid fragments from different sources. The method allows the unambiguous identification of a trait-associated haplotype over large stretches of DNA up to several kilobases. The invention can be used to identify haplotypes related to various conditions or diseases, in particular to the ability of a subject to respond to therapeutic treatments.

## Description

### FIELD OF INVENTION

The present invention relates to the field of genomics and genetic analysis, more particularly to genetic mapping of complex quantitative and qualitative traits. This invention more particularly relates to compositions and methods to identify haplotypes of associated allelic variants in nucleic acid fragments from different sources. The method allows the unambiguous identification of a trait-associated haplotype over large stretches of DNA up to several kilobases. The invention can be used to identify haplotypes related to various conditions or diseases, in particular to the ability of a subject to respond to therapeutic treatments.

### BACKGROUND

A major challenge for biology and medicine today is the identification of genes implicated in common, complex, human diseases like asthma, type 2 diabetes mellitus, obesity etc. The identification of such genes is usually carried out performing linkage and/or association studies in large family or patient samples. These studies can be performed using a variety of genetic markers (sequences in the genome which differ between individuals i.e. that are polymorph). The most widespread polymorphisms used are microsatellite markers consisting of short, specific repeat sequences or single nucleotide polymorphisms (SNP's) that differ in just one nucleotide. Different analysis technologies have been developed to genotype these markers like, gel-based electrophoresis, DNA hybridisation to an ordered array, identification using mass spectrometry.

The major goal of genetics is to link a phenotype (i.e. a qualitative or quantitative measurable feature of an organism) to a gene or a number of genes. Whatever the approach is, genetic studies are based on polymorphisms, i.e. base differences in the DNA sequence between two individuals at the same genetic locus. The existence of sequence differences for the same genetic locus is called allelic variation. It has long been known that different alleles of a gene can result in different expression of a given phenotype.

One approach to genetic analysis relies on association studies. Association studies follow the evolution of a given allele in a population. The underlying assumption is that at a given time in evolutionary history one polymorphism became fixed to a phenotype because:
a) it is itself responsible for a change in phenotype or;
b) it is physically very close to such an event and is therefore rarely separated from the causative sequence element by recombination (one says the polymorphism is in linkage disequilibrium with the causative event). The markers of choice for these studies are accordingly single nucleotide polymorphisms (SNP's). These polymorphisms show a simple base exchange at a given locus (i.e. they are bi- rarely tri-allelic). Association studies can be carried out either in population samples (cases vs. controls) or family samples (parents and one offspring where the transmitted alleles constitute the "cases" and the non-transmitted the "controls").

It has been proposed to correlate genomic variability with phenotypic traits e.g. drug response, by analysing individual SNPs. This individual SNP approach, however, requires thousands of patients and complex statistical analysis to detect possible predictive markers and may lead to a large number of markers that, upon subsequent testing, may not correlate with the phenotype of interest. Classical population geneticists created the term haplotype to describe the physical organization of genetic variation as it occurs on each pair of chromosomes in an individual. The haplotype is a set of alleles that are not separated by recombination and that are transmitted as a block. At the molecular level, a haplotype consists of multiple individual SNPs that are organized into one of the limited number of combinations that actually exist as units of inheritance.

Each haplotype, therefore, contains significantly more information than individual, unorganised SNPs. As a result, fewer patients are needed to detect a statistically significant correlation with a drug response (or any other particular trait) if haplotypes are used rather than individual, unorganized SNPs. As an example there may be 3 SNPs in a gene that slightly change the expression of the gene in a phenotypically imperceptible manner. However, the combination of the 3 SNPs (i.e. a haplotype consisting of these 3 SNPs on the same chromosomal arm) may produce a measurable phenotype.

To use this additional information it is essential to infer from the phase-unknown individual genotypes in a sample drawn from a population the haplotype frequencies in the population and the underlying haplotype pairs in the sample in order to find disease predisposing genes by some association or haplotype sharing algorithm. It has been shown in several studies that haplotypes can explain phenotypic effects where simple SNP typing failed to show any differences.

As any diploid individual has two of each existing chromosomes (one from the father one from the mother) it may be heterozygous for any typed variation and it is not possible to unambiguously determine the phase (i.e. the haplotype) for a number of polymorphisms that have been typed over some chromosomal region. Usually haplotype frequencies and haplotype pairs are estimated statistically via a maximum likelihood approach by a well-known expectation maximization (EM) algorithm, adapting it to a large number (up to 30) of biallelic loci (SNP), and including nuclear family information, if available, into the analysis. Parents are treated as an independent sample from the population.

### SUMMARY OF THE INVENTION

The present invention now provides novel genetic analysis methods to distinguish haplotypes in genomic fragments that overcome the drawbacks of the commonly used technologies based on cell fusion hybrids or statistical methods. It is especially advantageous as it allows:
a) the identification of haplotypes in pre-defined genomic fragments/genes or genome-wide;
b) the identification of haplotypes over several kilobases of DNA; and
c) the parallel typing of such haplotypes in a pool of several individuals with a phenotype of interest.

In a particular aspect, the present invention relates to a method to identify or determine haplotypes of associated allelic variants related to a particular trait. The method typically comprises (a) providing at least two populations of nucleic acid fragments from organisms having a common particular trait, (b) isolating nucleic acid fragments that are identical between said populations and (c) identifying or determining, from the isolated identical fragments, haplotypes of associated allelic variants, said haplotypes being related to said particular trait.

The invention can be applied to various nucleic acid populations originating from diverse sources, particularly human individuals. The invention may be used to determine haplotype related to various traits, such as a disease, a drug-response, a toxicity, etc.

The invention also relates to methods for evaluating the response of a subject to a particular therapeutic treatment comprising determining the presence or absence, in the genomic DNA from said subject, of a haplotype of associated allelic variants identified by a method as described above, said haplotype being characteristic of subjects that respond or that do not respond to said particular treatment.

The invention also encompasses compositions, kits and tools used to perform the above methods.

### DETAILED DESCRIPTION OF THE INVENTION

As indicated above, the present invention provides a method to identify or determine (or characterize) a haplotype of associated allelic variants related to a particular trait comprising (a) providing at least two populations of nucleic acid fragments from organisms having a common particular trait, (b) isolating nucleic acid fragments that are identical between said populations and (c) identifying or determining, from the isolated identical fragments, at least one haplotype of associated allelic variants, said haplotype being related to said particular trait.

In a specific embodiment, the invention relates to a method for the identification (or isolation or separation) of haplotype of associated allelic variants related to a particular trait from a mixture of at least two nucleic acid populations, comprising: a) separate digestion of the nucleic acids of said at least two populations with at least one restriction enzyme; b) ligation of specific adaptor sequences to the restriction fragments; c) amplification of the adaptor-ligated restriction fragments generated in a) and b) using adaptor-specific primers ; d) hybridisation of the amplification products from the different nucleic acid populations with each other and e) identification (or isolation or separation) of identical, fully matched, heterohybrid fragments constituting a haplotype.

The invention can be applied to various nucleic acid populations originating from diverse sources, particularly human individuals. In particular, the nucleic acid populations may be DNA populations, particularly DNA fragments, typically genomic DNA fragments. Typically, the nucleic acid populations are genomic DNA, in particular mammalian genomic DNA such as human genomic DNA. In a preferred embodiment, the nucleic acid populations are human genomic DNA from different subjects that share a trait of interest, in particular a phenotype or pathology. In this embodiment, the method of the present invention is directed at identifying haplotypes of the pathology, or involved in the response to pharmacological treatment.

The nucleic acid populations may also be genomic DNA from other sources, including prokaryotic (bacteria, pathogenic organisms, etc.), lower eukaryotic (yeasts, etc.), plants, viruses, and the like.

The nucleic acid population may comprise nucleic acid fragments of various size, which may be homogenous or heterogeneous. Typically, the nucleic acid populations comprise DNA fragments obtained by digestion of genomic DNA from distinct organisms with at least one restriction enzyme. The restriction enzyme may by any conventional enzyme, such as EcorI, EcoRV, HindIII, AatIII, etc. The choice of the enzyme can be made according to practical considerations, e.g., average size of the generated fragments, specificity for DNA species, enzymatic activity and ease of use, etc.

In this respect, in a particular embodiment, the nucleic acid populations are genomic DNA libraries from (mammalian, e.g., human) individuals being characterized by the presence and/or absence of a common trait. As indicated above, the term "individual" designates mammalians, e.g., humans, rodents, bovines, etc. as well as organisms from other species, including without limitation plants, cells, viruses, pathogens, etc.

While the nucleic acid population may comprise the total genomic DNA of a cell (or tissue or organism), or a complete genomic library, for instance, it should be noted that a screening or a selection of the starting nucleic acids might also be performed. In particular, the nucleic acid population may be a single specifically isolated DNA fragment or a pool of a selection of isolated fragments. The isolation of the specific DNA fragments may be performed for instance using any of the following methods:
a) direct isolation through microdissection of a chromosome;
b) cloning of specific fragments into an appropriate cloning vector (e.g. a plasmid).
c) amplification of specific genomic regions by means of polymerase chain reaction (PCR).

In this regard, in a particular embodiment, the nucleic acid fragments comprise a selected gene or gene fragment or genomic region. This may be done for instance where a particular trait is known to be correlated to a particular genomic region (e.g., a chromosome or a portion thereof). In such a case, the method would not necessarily be performed on total genomic libraries, but could be carried out using populations of nucleic acids comprising such particular region only. This may be applied to a particular gene or gene fragment as well.

In performing the instant invention, two or more nucleic acid populations can be used, originating from different sources. In preferred embodiments, 2 to 10 nucleic acid populations are used.

In a preferred embodiment of this invention, the nucleic acid populations comprise nucleic acid fragments that have been amplified.

The amplification may be performed in order to reduce the complexity of the starting material (as described above) and/or to facilitate the later processing of the samples and/or to obtain larger quantities of material. The amplification is thus performed separately from each source of material (i.e., the nucleic acid populations are nucleic acid fragments that have been amplified separately from individuals).

The amplification may be performed according to several methodologies.

In a first embodiment, a direct amplification is performed using locus-specific oligonucleotides as primers. Such locus-specific oligonucleotides may be chosen by the skilled person depending on the situation. They may also be (partially) random(ized) oligonucleotides.

In an other embodiment, the amplification is performed using primers that are specific for adaptor molecules added to (each or one) end of the nucleic acid fragments. In this embodiment, the amplification thus comprises :
- ligating specific adaptor sequences to the nucleic acid fragments, and
- amplifying the adaptor-ligated fragments using adaptor-specific primers.
   Indeed, a particular aspect of this invention resides in the use of adaptor molecules that facilitate specific amplification of the nucleic acids and specific treatment of the samples to increase the selectivity of the identification method.

Adaptor molecules are preferably short double stranded DNA fragments with known sequence composition. More preferably, the adaptor molecules are 5-100 base pair long double stranded DNA molecules, even more preferably 5-50 base pair long. The adaptor molecules allow the introduction of sequence features that greatly improve the genetic analysis procedure. More particularly, the introduction of these adaptors has the following advantages:
- the DNA can be amplified by PCR prior to the genetic analysis procedure allowing starting off with less material. Only one amplification per experiment, using a single primer sequence is necessary, making this method cheap;
- the adaptor may comprise features that allow the distinction of different DNA populations in a mixture;
- the adaptor may also provide a protection against enzymatic digestion of heterohybrid fragments created between DNAs from different individuals;
- the adaptor sequence is preferably designed to include a *mut H* or *mut* HL recognition sequence (e.g., GATC), allowing all mismatched fragments to be removed from the mixture, thereby increasing the selectivity and reducing the background signal;
- the adaptor molecule may also comprise a recognition site for a restriction enzyme that creates 3' sticky ends, such as *Aat* III.

In a preferred embodiment, the adaptor molecule is a 5-100 base long (double-stranded) oligonucleotide comprising at least one GATC motif. In a further preferred embodiment, each set of adaptors comprises a unique terminal sequence, preventing heterohybrids to form sticky ends.

The adaptor molecules can be prepared according to conventional techniques (artificial synthesis) and ligated to the restriction fragments (or to the nucleic acid population, where no restriction step is conducted), by conventional methods (using for instance a ligase enzyme, such as T4 ligase). The method of this invention preferably comprises the ligation of the nucleic acids to adaptor molecules resulting in DNA fragments that carry an adaptor sequence at both ends.

Another advantageous embodiment of the instant invention resides in the use of particular primers for the amplification reaction. The primers are preferably complementary to at least part of the adaptor molecule (or selected locus). The primers can be any oligonucleotide, preferably having 5 to 30 bases, even more preferably 5-20 bases. The portion of the primer that is complementary to the (portion of the) adaptor molecule (or selected locus) should preferably comprise at least 5, more preferably at least 10 bases, to ensure sufficient selectivity. Primers can be produced by the skilled person according to conventional techniques known in the art (preferably artificial nucleic acid synthesis).

In a preferred embodiment, the primers are labelled, which provides further advantages to the present method. In particular, the introduction of labelled primers for (PCR) amplification allows to distinguish the different DNA populations that are mixed. Indeed, the primer used to amplify each nucleic acid population may exhibit a different label, such as different unique 5' sequences (or some may be labelled and some not), allowing distinguishing the amplified products from each source. This avoids the need for any methylation step. Accordingly, no methylation-specific restriction enzymes are needed and a significant decrease of the cost per experiment can be obtained. Furthermore, the use of labelled primers makes it possible to carry out more than pair-wise comparisons (several individuals included in a reaction, i.e., more than two nucleic acid populations). This can be used to increase the resolution of the method (smaller IBD regions are detected).

Moreover, the primers can be designed in a way that allows an exonuclease to attack homoduplexes formed upon hybridisation between the nucleic acid populations, but not the heteroduplexes. Accordingly, the restriction ends play no part in the choice of the restriction enzyme for digestion of the nucleic acid populations. The enzymes can thus be chosen according to practical considerations as discussed above (e.g., size of the generated fragments, specificity for DNA species, enzymatic activity and ease of use).

The adaptors or complementary primers can be labelled by (i) adding a unique 5'-sequence to each oligonucleotide (adaptor or primer), (ii) adding a chemical activity to the oligonucleotide which provides a means to distinguish between the products from different DNA sources and (iii) adding modified nucleotides into the oligonucleotides allowing to distinguish between the products from different DNA sources. Preferred labelling technique comprises the introduction of a unique 5' sequence to each set of primers.

Amplification of the nucleic acids (or restriction fragments) may be accomplished by polymerase chain reaction (PCR), according to conventional techniques. Preferably, the amplification is carried out by polymerase chain reaction using a high fidelity, long-range DNA polymerase. Examples of such polymerases include *Pfx* polymerase (Life Technologies), *Z-Taq* polymerase (TaKaRa), etc. Several amplification cycles may be performed, more particularly from 25 to 40.

The isolation (or separation) of nucleic acid fragments that are identical between the above (amplified) populations can be performed in several ways. Preferably, the isolation (or separation) comprises (i) cross-hybridizing nucleic acid fragments from said at least two nucleic acid populations and (ii) isolating or separating fully matched heterohybrids from the hybridization mixture.

Cross hybridization is generally performed in liquid solution, in any appropriate buffer or suspension. The hybridization is carried out under high stringency conditions favoring the correct pairing of homologous DNA strands. Preferably the hybridization is carried out in a solution that is composed of an aquaous phase including formamide and an organic phase composed of phenol where hybridization takes place at the interface of those two phases under slightly denaturing conditions. An example for such an hybridization buffer is a solution composed of 2 mol/1 sodium thiocyanate, 10 mmol/1 Tris-HCl (pH 8.0), 0.1 mmol/1 EDTA and 8% formamide with an equal amount of water saturated phenol.

Preferably the two DNA populations are mixed in a 1:1 ratio with at least 1 µg of DNA from each population. If more than two populations are mixed, equimolar amounts are preferably used. Preferably the total amount of DNA does not exceed 10 µg of DNA. It should be understood that other specific conditions may be used, without deviating from the scope of the present invention.

The fully matched heterohybrids are preferably isolated or separated by (i) separating homohybrids from heterohybrids and (ii) (identification and) elimination of mismatched heterohybrids (hybrids between fragments with different haplotype composition).

The heterohybrids can be separated from the homohybrids based on labelling of oligonucleotides (adaptors or primers), as described above. In particular, the separation may be performed based on the use of oligonucleotides with a unique 5' end sequence for each nucleic acid population. According to this embodiment, homohybrids only will be blunt ended, i.e., comprise perfectly matched DNA ends (the unique 5' end sequence of the specific primer). Accordingly, all homohybrids can be eliminated by treatment of the hybridisation product with an enzyme that specifically digest blunt-ended double stranded DNA fragments, such as *Exo* III. Treatment with *Exo* III results in the formation of single-strands, which can be eliminated through various methods, such as through binding to a single strand-specific matrix.

In this regard, in a specific embodiment, the method of the present invention comprises a) separate amplification of the restriction fragments from different sources using a primer with a unique 5' sequence for each DNA source; b) mixing the amplification products from said different sources carrying unique 5' ends; c) denaturation and rehybridizing said DNA's; d) digesting perfectly matched (blunt ended) DNA's (homoduplexes) by *Exo* III and e) elimination of the *Exo* III created single strands through binding to a single strand specific matrix.

In another preferred embodiment the adaptor carries a unique 5' sequence for each DNA source and the procedure is altered to include the following steps a) ligation of the adaptor to the DNAs from different sources; b) mixing the products from said different sources carrying unique 5' ends; c) denaturation and rehybridizing said DNA's; d) digesting perfectly matched (blunt ended) DNA's (homoduplexes) by *Exo* III and e) elimination of the *Exo* III created single strands through binding to a single strand specific matrix.

Mismatched heterohybrids may be preferably eliminated with mismatch repair enzymes. In particular, the distinction between (or elimination or separation) of mismatched and perfectly matched nucleic acid fragments can be performed using mismatch repair enzymes *mut*S, *mut*L and/or *mut*H, or derivatives or homologues thereof. Derivatives include fragments or variants of the *Mut* proteins, i.e., any polypeptide or fragment derived there from and retaining the biological activity of the protein. Preferred derivatives retain at least 80% of the primary structure of the *Mut* protein. Homologues include proteins exhibiting the same type of enzymatic activity in other biological systems (yeasts, plants, etc.). Mut enzymes, homologues thereof and corresponding methods of preparation can be found, for instance in Su and Modrich (Proc Natl Acad Sci U S A. 1986 Jul; 83(14):5057-61.), Lahue et al., (Proc Natl Acad Sci U S A. 1987 Mar; 84(6):1482-6) or Bocker et al., (Cancer Res. 1999 Feb 15;59(4):816-22.). The sequence of these enzymes is also available on gene libraries.

In particular, mismatched nucleic acid fragments can be eliminated by (i) incubating the hybridisation mixture with *Mut*S (which binds mismatch) and contacting the resulting product with a *Mut*S-binding material (e.g., support, bead, column, etc.).

Mismatched nucleic acid fragments can also be eliminated by incubating the hybridisation mixture with *Mut*S, *Mut*L and *Mut*H, resulting in a specific cleavage of mismatched hybrids.

The identified (or separated or isolated) identical DNA fragments are further analysed to determine a haplotype of a gene or genomic fragment, and the like. More particularly, the fragments can be analysed by sequencing.

In a particular embodiment, the identifying or determining step (c) comprises sequencing all or a portion of at least one identical fragment obtained in step (b). More preferably, the sequence of the fragment is further compared to a reference sequence from an organism that does not exhibit said trait.

The invention can be used to identify haplotypes involved in pathologies, such as complex pathologies (obesity, asthma, cardiovascular diseases, CNS disorders, etc.) or any other phenotypic trait of interest, especially response of individuals to pharmacological treatment. In this regard, the starting nucleic acid populations may be obtained from organisms (or individuals) that (i) do not respond to a particular therapeutic treatment [including chemical, pharmaceutical, vaccinal, physical (e.g., radiation), etc treatments], or (ii) that do respond to a particular therapeutic treatment or (iii) that exhibit a common pathological condition. The organism is preferably a human subject. The therapeutic treatment includes, for instance, anticancer treatment, antiviral treatment, etc. Most preferably, the at least two nucleic acid populations are from unrelated human subjects.

A further object of this invention also resides in methods for evaluating the response of a (human) subject to a particular therapeutic treatment, comprising determining the presence or absence, in the genomic DNA from said subject, of a haplotype of associated allelic variants identified by a method as described above, said haplotype being characteristic of subjects that respond or that do not respond to said particular treatment.

Typically, determining the presence or absence of said haplotype comprises (i) hybridizing genomic DNA from said subject with a labeled probe comprising said haplotype and/or (ii) sequencing genomic DNA from said subject.

The invention also encompasses kits, compositions, genetic constructs and the like that are used to implement the above methods, such as primers, adaptors, fusion molecules, kits of reagents etc.

Further aspects and advantages of the present invention will be disclosed in the following experimental section, which should be regarded as illustrative and not limitative. All references cited in this document are incorporated therein by reference.

### EXAMPLES

### Example 1 : Establishing a haplotype for a specific gene

Genomic DNA from different sources (at least 2) from individuals sharing a common phenotype of interest are (e.g. non-responders to a specific drug) is amplified for a region harbouring a gene believed to be implicated in the phenotype for which an initial SNP map exists or has been established. This amplification is carried out using specific primers that allow the amplification of fragments up to 6 or 7 kilobases in length. The primers used for the amplification of each individual are composed of two parts: a) a sequence specific for the genomic fragment to be amplified common to the primers for all DNAs and a unique end-sequence of maximally 6 bases specific for each DNA. Each DNA is amplified separately and the resulting amplicons are pooled after the reaction. The amplicons are then denatured and renatured under stringent conditions to produce heterohybrids between the different DNAs. The DNA mixture is then incubated with an exonuclease that will attack the blunt ended homohybrids but leave intact the sticky-ended (or forked) heterohybrids. The long single stranded fragments from the homohybrids thus created are eliminated using a single strand specific matrix (e.g. BNDC). In the following steps the heterohybrids are subjected to treatment with the E.coli mismatch repair enzymes mutS, mutL and mutH. Any fragments containing mismatches (i.e. fragments not being 100% identical and therefore representing different haplotypes) are nicked by mutH, the nicked sites are widened using an exonuclease (e.g. exo III) and the single stranded fragments are then eliminated as described above. The resulting products constituting perfect haplotypes can then be subjected to common SNP genotyping procedures to identify the respective haplotypes. In cases where more than one haplotype is present the selected fragments may be subjected to a sub-cloning step in an appropriate vector to produce a library of haplotypes that can then be genotyped.

## Claims

1. A method to identify or determine haplotypes of associated allelic variants related to a particular trait, comprising (a) providing at least two populations of nucleic acid fragments from organisms having a common particular trait, (b) isolating nucleic acid fragments that are identical between said populations and (c) identifying or determining, from the isolated identical fragments, haplotypes of associated allelic variants, said haplotypes being related to said particular trait.

2. The method of claim 1, wherein the nucleic acid populations comprise DNA fragments.

3. The method of claim 2, wherein the nucleic acid populations comprise genomic DNA fragments.

4. The method of claim 3, wherein the nucleic acid populations comprise DNA fragments obtained by digestion of genomic DNA from said organisms with at least one restriction enzyme.

5. The method of any one of the preceding claims, wherein the nucleic acid fragments comprise a selected gene or gene fragment.

6. The method of any one of the preceding claims, wherein the nucleic acid populations are genomic DNA libraries from individuals being **characterized by** the presence and/or absence of a common trait.

7. The method of any one of the preceding claims, wherein the nucleic acid populations are nucleic acid fragments that have been amplified (e.g., by polymerase chain reaction) separately from individuals having a common trait.

8. The method of claim 7, wherein the fragments are amplified using locus-specific oligonucleotides as primers.

9. The method of claim 8, wherein the primer sequences comprise a label.

10. The method of claim 7, wherein said amplification comprises
- ligating specific adaptor sequences to the nucleic acid fragments, and
- amplifying the adaptor-ligated fragments using adaptor-specific primers.

11. The method of claim 10 wherein the adaptor sequences comprise a label.

12. The method of claim 10 or 11, wherein the adaptor sequences comprise a recognition site for mut H.

13. The method of any one of the preceding claims, wherein step (b) comprises (i) cross-hybridizing nucleic acid fragments from said at least two nucleic acid populations and (ii) isolating or separating fully matched heterohybrids from the hybridization mixture.

14. The method of claim 13, wherein the fully matched heterohybrids are isolated or separated by (i) separating homohybrids from heterohybrids and (ii) elimination of mismatched heterohybrids.

15. The method of claim 14, wherein mismatched heterohybrids are eliminated with mismatch repair enzymes.

16. The method of any one of the preceding claims, wherein identifying or determining haplotypes comprises sequencing all or a portion of at least one identical fragment obtained in step (b).

17. The method of claim 16, wherein the sequence of the fragment is further compared to a reference sequence from an organism that does not exhibit said trait.

18. The method of any one of the preceding claims, wherein the at least two nucleic acid populations are from human subjects that do not respond to a particular therapeutic treatment.

19. The method of any one of claims 1 to 17, wherein the at least two nucleic acid populations are from human subjects that respond to a particular therapeutic treatment.

20. The method of any one of claims 1 to 17, wherein the at least two nucleic acid populations are from human subjects that exhibit a common pathological condition.

21. The method of any one of the preceding claims, wherein the at least two nucleic acid populations are from unrelated human subjects.

22. A method for evaluating the response of a subject to a particular therapeutic treatment comprising determining the presence or absence, in the genomic DNA from said subject, of a haplotype of associated allelic variants identified by a method of any one of claims 1 to 17, said haplotype being characteristic of subjects that respond or that do not respond to said particular treatment.

23. The method of claim 22, wherein determining the presence or absence of said haplotype comprises (i) hybridizing genomic DNA from said subject with a labeled probe comprising said haplotype and/or (ii) sequencing genomic DNA from said subject.
